# EUROPÄISCHE PATENTSCHRIFT

(11) **EP 0 649 642 B1**
(45) Veröffentlichungstag und Bekanntmachung des Hinweises auf die Patenterteilung: **09.12.1998**
(21) Anmeldenummer: 93810738.0
(22) Anmeldetag: 21.10.1993
(51) Int. Cl.: A61F 2/36

(54) **Bausatz für eine modulare Femurkopfprothese, insbesondere eine Reoperationsprothese, und Femurkopfprothese aus einem derartigen Bausatz**
Construction kit for a modular femoral head prosthesis, especially a reoperation prosthesis, and femoral head prosthesis made from such a kit
Eléments de construction pour une prothèse de tête fémorale modulaire, en particulier une prothèse de réopération, et prothèse de tête fémorale obtenue avec de tels éléments

(43) Veröffentlichungstag der Anmeldung: 26.04.1995
(60) Teilanmeldung: 98104084.3
(73) Patentinhaber: Sulzer Orthopädie AG, 6340 Baar (CH)
(72) Erfinder: Baur, Nikolaus, CH-8932 Mettmenstetten (CH); Lamprecht, Stefan, CH-8303 Bassersdorf (CH)
(74) Vertreter: Trieblnig, Adolf

(56) Entgegenhaltungen:
- EP-A- 0 019 042
- EP-A- 0 304 756
- EP-A- 0 399 920
- DE-A- 2 933 230
- DE-A- 3 033 227
- DE-A- 3 736 304
- DE-A- 4 106 876
- DE-C- 211 137
- DE-C- 3 805 303
- FR-A- 2 605 514
- FR-A- 2 622 791
- FR-A- 2 633 509
- US-A- 4 051 559

## Beschreibung

Die Erfindung betrifft einen Bausatz für eine modulare Femurkopfprothese, insbesondere eine Reoperationsprothese, enthaltend einen in einem Femurknochen verankerbaren distalen Schaftteil und einen auf dessen proximaler Endpartie mittels einer Schraubverbindung befestigbaren, mit einer künstlichen Gelenkkugel bestückbaren Halsteil.

Ferner betrifft die Erfindung eine aus einem derartigen Bausatz zusammengesetzte Femurkopfprothese.

US-A-4051559 und FR-A-2605514 offenbaren modulare Femurkopfprothesen laut Oberbegriff des Anspruchs 1.

Eine aus der EP 0 399 920 A1 bekannte Femurkopfprothese der genannten Art enthält einen mit einem Aussengewinde versehenen Schaftteil mit einem kegelstumpfförmigen proximalen Fortsatz und einen auf diesen aufsetzbaren Halsteil mit einem gegenüber der Längsachse des Schaftteils abgewinkelten Aufsteckdorn für die Gelenkkugel. Der Halsteil ist durch eine in eine axiale Bohrung des Schaftteils oder eines damit verbunden Einsatzteils einschraubbare Sicherungsschraube gegen den Fortsatz des Schaftteils verspannbar gehalten. Der Schaftteil der bekannten Endoprothese ist in einem Längenabschnitt seiner axialen Bohrung mit zungenartig ausstellbaren Segmenten ausgeführt, welche über einen in axialer Richtung in die Bohrung einführbaren Verstellteil mit keilförmigen Abstützflächen je gegenüber zwei benachbarten feststehenden Segmenten des Schaftteils radial nach aussen gegen die den Schaftteil umgebende Partie des Femurknochens verspannbar sind. Die auf den Femurknochen zu übertragenden Hauptbelastungskräfte werden jeweils über die gegenüber der Längsachse des Schaftteils lateral versetzte Gelenkkugel in die Femurschaftprothese eingeleitet. Entsprechend werden Femurschaftprothesen der genannten Art jeweils durch relativ grosse dynamische Kräfte und Biegemomente beansprucht. Derartige dynamische Beanspruchungen können bei bisherigen Endoprothesen der genannten Art zu einer Lockerung der Schraubverbindung zwischen Halsteil und Schaftteil und/oder einer Lockerung der Verankerung des Schaftteils im Femurknochen und damit, z.B. im Bereich einer dübelartigen Aufspreizung des Schaftteils, zu hohen örtlichen Beanspruchungen des Knochengewebes führen.

Der Erfindung liegt die Aufgabe zugrunde, einen insbesondere in dieser Hinsicht verbesserten Bausatz für eine Femurschaftprothese der genannten Art in einer einfachen, robusten Bauweise zu schaffen, welcher aus wenigen, günstig beanspruchten Bauteilen besteht und welcher eine von den dynamischen Beanspruchungen im Implantationsbereich unbeeinflusste, bleibende Verbindung zwischen dem Halsteil und dem Schaftteil sowie zwischen dem Halsteil und dem Femurknochen gewährleistet.

Diese Aufgabe wird erfindungsgemäss durch den Bausatz laut Anspruch 1 und durch die Femurkopfprothese laut Anspruch 9 gelöst.

Der erfindungsgemäss ausgebildete Bausatz gestattet eine funktionelle Trennung der formschlüssigen, winkelgerechten Positionierung des Halsteils auf dem Schaftteil einerseits und der kraftschlüssigen Verbindung zwischen dem Halsteil und dem in den Femurknochen eingesetzten Schaftteil andererseits. Entsprechend können die Abstützflächen des Halsteils und des Schaftteils für die Uebertragung der einem vorbestimmten, grössten Biegemoment entsprechenden Vorspannkraft der Dehnschaftschraube, und die Führungsflächen für die Uebertragung relativ geringer Querkräfte dimensioniert werden. Ein weiterer Vorteil der erfindungsgemässen Ausführung besteht darin, dass durch die über die Dehnschaftschraube gegeneinander verspannbaren, mit hoher Flächenpressung aneinanderliegenden Abstützflächen des Halsteils und des Schaftteils ein Austreten von zwischen diesen Teilen entstehendem Abrieb verhindert wird. Der erfindungsgemässe Bausatz kann aus relativ wenigen Bauteilen bestehen, welche einem Vorrat von mehreren, mit unterschiedlichen Querschnitten und/oder unterschiedlichen, z.B. zentimeterweise abgestuften Längenabmessungen ausgeführten Schaftteilen und von mehreren, z.B. je in zwei, mit unterschiedlichen Abmessungen ausgeführten Halsteilen und Dehnschaftschrauben entnommen und zu einer den gegebenen anatomischen Verhältnissen entsprechenden Femurkopfprothese zusammengesetzt werden können.

In den abhängigen Patentansprüchen sind vorteilhafte Ausgestaltungen des Erfindungsgegenstandes angegeben.

Weitere Merkmale und Einzelheiten ergeben sich aus der folgenden Beschreibung in Form in der Zeichnung schematisch dargestellten Ausführungsbeispielen nach der Erfindung, in Verbindung mit den Patentansprüchen. In der Zeichnung zeigen:
- Fig. 1: Eine erfindungsgemäss ausgebildete Femurkopf-Endoprothese in einer Teilansicht mit Teilschnitt;
- Fig. 2: die Endoprothese in einem Längsschnitt entsprechend der Linie II-II in Fig. 1;
- Fig. 3: die Endoprothese in einer Draufsicht;
- Fig. 4: die Endoprothese in einem Querschnitt entsprechend der Linie IV-IV in Fig. 1;
- Fig. 5: einen Querschnitt entsprechend der Linie V-V in Fig. 1;
- Fig. 6: eine Endoprothese in einer abgewandelten Ausführungsform, in einer der Fig. 2 entsprechenden Darstellung.

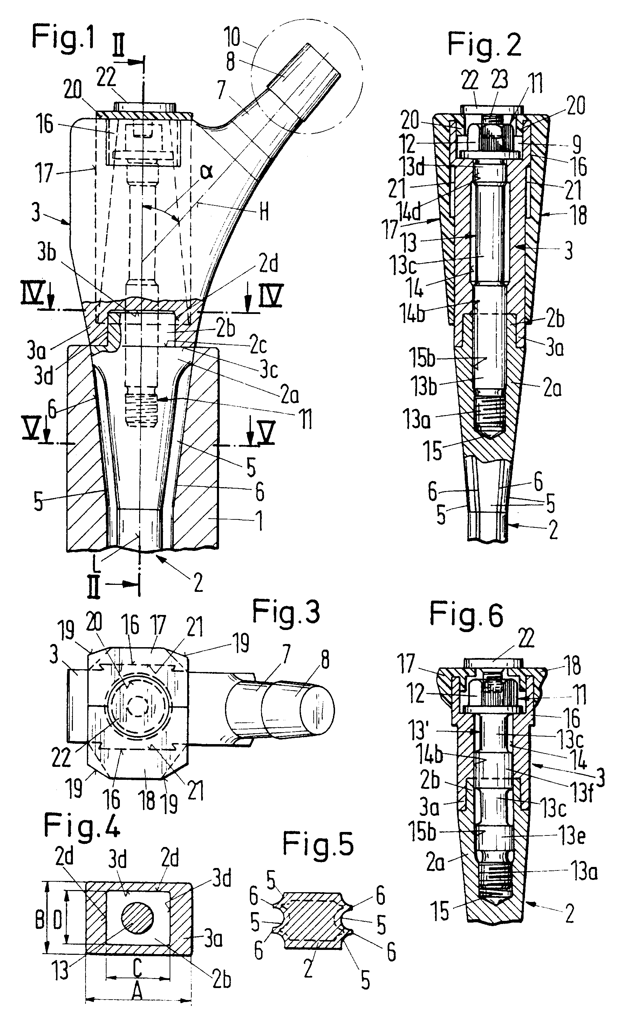

Die Femurkopfprothese nach den Figuren 1 bis 5 ist Teil einer Reoperationsprothese, die als Zweit- oder Drittprothese zur Verwendung bei Patienten vorgesehen ist, bei denen eine entsprechende, früher eingesetzte Endoprothese aus irgendwelchen Gründen, z.B. wegen Lockerung ihrer Verankerung im Knochengewebe, reoperiert und ersetzt werden muss. Die dargestellte Femurkopfprothese enthält einen in einen Femurknochen 1 implantierbaren und in diesem mittels Knochenzement oder, wie dargestellt, zementfrei verankerbaren distalen Schaftteil 2 und einen auf dessen proximale Endpartie 2a aufsetzbaren Halsteil 3 aus einem körperverträglichen Metall, z.B. Titan. Der Halsteil 3 ist als Ersatz für eine fehlende Knochenpartie, beim dargestellten Beispiel die proximale Endpartie des Femurknochens 1 vorgesehen, welche, insbesondere in Reoperationsfällen, etwa infolge von Abbauerscheinungen und Demineralisation des Knochengewebes häufig teilweise oder, wie dargestellt, vollständig reseziert werden muss. Der Schaftteil 2 enthält einen in Richtung seiner Längsachse L in den Femurknochen 1 einsetzbaren, von seiner Endpartie 2a gegen seine nicht dargestellte distale Endpartie konisch sich verjüngenden Schaftkörper und einen an der Endpartie ausgebildeten zapfenartigen Fortsatz 2b, der in eine entsprechende, am Halsteil 3 ausgebildete Aussparung 4 vorsteht. Der Schaftkörper 2 ist mit mehreren, zumindest über einen Teil seiner Längserstreckung verlaufenden Längsnuten 5 bzw. zwischen diesen ausgebildeten, in das Knochengewebe eindringenden schneidenartigen Längsrippen 6 ausgeführt und durch diese im Femurknochen 1 gegen Verdrehen gesichert gehalten.

Der Halsteil 3 enthält einen dem resezierten Ende des Femurknochens 1 entsprechenden Hauptkörper mit einer auf die Endpartie 2a des Schaftteils 2 aufsetzbaren Anschlusspartie 3a und einem seitlich angeformten, bezüglich der Längsachse L des Schaftteils 2 lateral abstehend positionierbaren Aufsteckdorn 7, dessen Längsachse H unter einem Winkel α zur Längsachse L geneigt ist. Der Aufsteckdorn 7 endet in einem Konus 8, auf den in bekannter Weise eine gegenüber der Längsachse lateral versetzte, strichpunktiert dargestellte künstliche Gelenkkugel 10 aufsetzbar ist. Die Endpartie 2a des Schaftteils 2 und zumindest die Anschlusspartie 3a, darstellungsgemäss der ganze Hauptkörper, des Halsteils 3 sind je mit einem zumindest annähernd rechteckförmigen Querschnitt und mit entsprechenden, quer zur Längsachse L gestellten, gegeneinander abstützbaren rechteckförmigen Abstützflächen 2c bzw. 3c ausgeführt, welche je mit in lateraler Richtung orientierter grosser Längenabmessung A und in transversaler Richtung orientierter kleinerer Breitenabmessung B angeordnet sind. Der Fortsatz 2b des Schaftteils 2 und die Aussparung 3b des Schaftteils 3 sind ebenfalls je mit einem entsprechenden, annähernd rechteckförmigen Querschnitt und mit in Richtung der Längsachse L formschlüssig zusammenführbaren Mantelflächen 2d bzw. 3d ausgeführt, durch welche der Schaftteil 2 und der Halsteil 3 in einer bezüglich der Lage der Gelenkkugel 10 definierten Stellung miteinander kuppelbar sind.

Der Halsteil 3 ist auf dem Schaftteil 2 mittels einer Dehnschaftschraube 11 befestigt, welche einen in einer Vertiefung 9 des Halsteils 3 versenkt abstützbaren Kopf 12 und einen Schraubenschaft 13 aufweist, der eine Bohrung 14 des Halsteils 3 und eine axiale Bohrung 15 des Schaftteils 2 durchsetzt. Der Schraubenschaft 13 ist mit einem in eine Gewindebohrung des Schaftteils 2 einschraubbaren Gewindeabschnitt 13a und zwei gegenüber diesem verdickten, im Abstand voneinander angeordneten Führungsabschnitten 13b und 13d sowie mit einem diese verbindenden, elastisch vorspannbaren Dehnabschnitt 13c ausgeführt, der in bekannter Weise einen Querschnitt aufweist, der kleiner ist als der Kernquerschnitt des Gewindeabschnitts 13a. An den Führungsabschnitten 13b und 13d sind zylindrische Passflächen ausgebildet, welche zum Zusammenwirken mit entsprechenden, an den Bohrungen 14 und 15 ausgebildeten Passflächen bestimmt sind. Gemäss Fig. 2 ist der Führungsabschnitt 13d in der Nähe des Kopfes 13 ausgebildet und wirkt mit einem Bohrungsabschnitt 14d des Halsteils 3 zusammen, während der Führungsabschnitt 13b sich über die Trennstelle zwischen dem Halsteil 3 und dem Schaftteil 2 erstreckt und mit einem Bohrungsabschnitt 14b des Halsteils 3 und einem Bohrungsabschnitt 15b des Schaftteils 2 zusammenwirkt.

Der Halsteil 3 und der Schaftteil 2 sind durch Anziehen der Dehnschaftschraube 11 gegeneinander mit einer Vorspannkraft verspannbar, welche entsprechend einem vorbestimmten grössten Biegemoment aus der am Halsteil 3 gegenüber der Längsachse L lateral versetzt angreifenden, über die Gelenkkugel 10 eingeleiteten Hauptbelastungskraft gewählt werden kann. Die jeweilige Vorspannkraft ist durch das Anzugsdrehmoment der Dehnschaftschraube 11 bestimmt, welches in bekannter Weise an einem Drehmomentenschlüssel auf einen vorbestimmten Höchstwert begrenzt werden kann. Die der Vorspannkraft entsprechende Stützkraft wird ausschliesslich durch die den Fortsatz 2b und die Aussparung 3b umgebenden rechteckförmigem Abstützflächen 2c bzw. 3c übertragen, welche dementsprechend für die Aufnahme der aus der Hauptbelastung resultierenden dynamischen Kräfte und Biegemomente dimensioniert sind.

Beim Aufsetzen des Halsteils 3 auf den Schaftteil 2 werden diese Teile durch die Führungsflächen 2d und 3d des Fortsatzes 2b bzw. der Aussparung 3b zentriert und gegen Verdrehen gesichert. Durch die Dehnschaftschraube 11 wird eine zusätzliche Feinzentrierung des Halsteils 3 und des Schaftteils 2 über die zusammenwirkenden Passflächen der Führungsabschnitte 13b und 13d und der Bohrungsabschnitte 14b und 15b bzw. 14d erzielt, welche zugleich eine von Biegebeanspruchungen freie Führung des Dehnabschnitts 13c der Dehnschaftschraube 11 gewährleisten. Durch die beschriebene Anordnung, welche eine funktionelle Trennung der die Längskräfte übertragenden Teile und der die Querkräfte übertragenden Teile ermöglicht, wird eine formstabile, biegesteife Verbindung zwischen dem in sich biegesteifen Halsteil 3 und dem biegesteifen Endabschnitt des für die Einleitung der zu übertragenden Kräfte in lateraler Richtung verbreiterten des Schaftteils 2 erzielt. Durch diese Ausführung wird auch bei hohen wechselnden Beanspruchungen eine sichere Uebertragung der zwischen der Gelenkkugel 10 und dem Schaftteil 2 wirkenden dynamischen Kräfte und Biegemomente gewährleistet.

Die innerhalb der Aussparung 3b formschlüssig zusammenführbaren Führungsflächen 2d und 3d sind durch die sie umgebenden, unter Vorspannung zusammengepressten Abstützflächen 2c und 3c nach aussen abgedichtet, so dass ein Austreten von gegebenenfalls entstehendem Abrieb in das umgebende Gewebe sicher verhindert werden kann. Durch die für die Aufnahme des grössten Biegemomentes optimierbaren, in Form rechteckiger Hohlquerschnitte ausgeführten Abstützflächen 2c und 3c des Schaftteils 2 und des Halsteils 3 - mit den für das Widerstandsmoment des Querschnitts massgebenden Flächenteilen in den lateralen Randpartien - kann zugleich die bei der Uebertragung der Druckkräfte auftretende Beanspruchung auf Flächenpressung innerhalb vorbestimmter Grenzen gehalten werden. Um eine für unterschiedliche anatomische Verhältnisse geeignete, möglichst universell einsetzbare Ausführung zu erhalten, können die Abstützflächen 2c und 3c je z.B. eine Längenabmessung A = ca. 20 mm bis 30 mm und eine Breitenabmessung B = ca. 15 mm bis 20 mm aufweisen. Der Fortsatz 2b und die Aussparung 3b können entsprechend je z.B. eine Längenabmessung C = ca. 10 bis 20 mm und eine Breitenabmessung D = ca. 10 bis 15 mm aufweisen. Es versteht sich, dass auch andere, den jeweiligen anatomischen Verhältnissen angepasste Abmessungen der Endpartie 2a und der Anschlusspartie 3a möglich sind.

Als Ersatz für eine fehlende, resezierte Knochenpartie können an den Seitenwänden des Halsteils 3, darstellungsgemäss anterior und posterior, backenartige Füllkörper 17 bzw. 18 angebracht werden. Diese können darstellungsgemäss mit abgeschrägten Eckpartien 19 und unterschiedlichen, gegen den Halsteil 2 hin abnehmenden Dicken ausgeführt sowie mit nicht dargestellten Mitteln zum daran Befestigen von Muskeln und Bändern versehen sein. Die Füllkörper 17 und 18, die aus Metall oder, wie dargestellt, aus einem körperverträglichen Kunststoff bestehen können, sind je mit einer auf die proximale Endpartie des Halsteils 3 aufsetzbaren und in deren Vertiefung 9 einführbaren, halbkreisförmigen Kragenpartie 20 und einer nutenartigen Führungsbahn 21 ausgeführt, mit der der betreffende Füllkörper 17 bzw. 18 auf eine an der Seitenwand des Halsteils 3 ausgebildete Führungspartie in Form eines schienenartigen Nockens 16 aufgesetzt und in Richtung der Längsachse L auf die betreffende Seitenwand aufgeschoben werden kann, bis die Kragenpartie 20 in die Vertiefung 9 einrastet. Der Nocken 16 und die Führungsbahn 21 können je mit einem rechteckigen oder, wie dargestellt, einem schwalbenschwanzförmigen Querschnitt ausgeführt sein. Nach einer anderen Ausführungsform können die Führungsbahnen 21 am Halsteil 3 und entsprechende Führungsteile an den Füllkörpern 17, 18 ausgebildet sein.

Zur axialen Sicherung der durch die Kragenpartie 20 zentrierten und durch die Nocken 16 seitlich geführten Füllkörper 17 und 18 kann ein die Vertiefung 9 überdeckender Deckel 22 vorgesehen sein, welcher mit einem in den Schraubenkopf 12 einschraubbaren Gewindezapfen 23 versehen und gegen die Kragenpartie 20 verspannbar ist. Der Deckel 22 verhindert zugleich ein Einwachsen von Gewebe in die Vertiefung 9. Die beschriebene Ausführung der Füllkörper 17 und 18 und des Deckels 23 gestattet dem Operateur die Auswahl der den jeweiligen anatomischen Verhältnissen entsprechenden Füllkörper 17, 18 aus einem Vorrat von in unterschiedlichen Ausführungen bereitgestellten Füllkörpern und die Anbringung der Füllkörper 17, 18 auf dem bereits implantierten Halsteil 3 während einer vorteilhaft späten Operationsphase.

Es ist auch eine Ausführung möglich, bei der nur einer der Füllkörper 17 bzw. 18 auf dem Halsteil 3 angebracht wird. Ferner kann ein entsprechend geformter Füllkörper auch an der lateralen Seitenwand des Halsteils 3 angebracht werden.

Die Ausführung nach Fig. 6 entspricht im wesentlichen der vorstehend beschriebenen Ausführung, wobei der Halsteil 3 eine geringere Bauhöhe und die Dehnschaftschraube 11 einen entsprechend kürzeren Schraubenschaft 13' als beim Beispiel nach den Figuren 1 und 2 aufweist. Der Schraubenschaft 13' ist mit zwei elastisch vorspannbaren Dehnabschnitten 13c und zwei Führungsabschnitten 13e und 13f ausgeführt, wobei der den Gewindeabschnitt 13a anschliessende Führungsabschnitt 13e ausschliesslich mit dem Bohrungsabschnitt 15b des Schaftteils 2 zusammenwirkt und der zwischen den beiden Dehnabschnitten 13c angeordnete Führungsabschnitt 13f sich über die Trennstelle zwischen dem Halsteil 3 und dem Schaftteil 2 erstreckt und mit dem oberen Ende des Bohrungsabschnitts 15b des Schaftteils 2 und dem unteren Ende des Bohrungsabschnitts 14b des Halsteils 3 zusammenwirkt. Entsprechend werden auch bei dieser Ausführung eine Feinzentrierung des Schaftteils 2 und des Halsteils 3 durch den Führungsabschnitt 13f und eine im wesentlichen biegungsfreie Beanspruchung der Dehnschaftschraube 11 erzielt.

Es sind zahlreiche abgewandelte Ausführungsformen der Erfindung möglich. Anstelle der dargestellten, für die Aufnahme der Biegebeanspruchungen besonders geeigneten Ausführung mit annähernd rechteckigen Abstützflächen 2c und 3c ist auch eine Ausführung mit entsprechenden, z.B. annähernd kreisförmigen oder ovalen Abstützflächen möglich. Ebenso können anstelle des dargestellten, rechteckförmigen Fortsatzes 2b und der entsprechenden Aussparung 3b ein entsprechender, z.B. zylindrischer oder sphärischer Fortsatz und eine entsprechende Aussparung sowie ein ausserhalb dieser Aussparung angeordnetes Zentrierelement vorgesehen sein, welches eine entsprechende winkelgetreue Positionierung des Halsteils 3 auf dem Schaftteil 2 ermöglicht.

Zusammenfassend lässt sich die Erfindung wie folgt beschreiben:

Der Bausatz enthält einen distalen Schaftteil 2 und einen mit einer Gelenkkugel 10 bestückbaren Halsteil 3, der auf dem Schaftteil 2 über quer zur Längsachse L des Schaftteils 2 gestellte, annähernd rechteckförmige Abstützflächen 2c bzw. 3c abgestützt ist. Der Schaftteil 2 und der Halsteil 3 sind mit parallel zur Längsachse L orientierten, formschlüssig zusammenführenden prismatischen Führungsflächen 2d bzw. 3d ausgeführt und über eine entsprechend einem aufzunehmenden, vorbestimmten Biegemoment elastisch vorspannbare Dehnschaftschraube 11 miteinander biegesteif verbunden. Die Führungsflächen 2d und 3d sind je an einem zapfenartigen Fortsatz 2b des Schaftteils 2 bzw. an einer entsprechenden Aussparung 3b des Halsteils 3 ausgebildet. Diese Ausführung gestattet eine formschlüssige Zentrierung und eine genaue Positionierung des Halsteils 3 auf dem Schaftteil 2 durch die Führungsflächen 2d, 3d und eine davon unabhängige kraftschlüssige Verbindung zwischen dem Halsteil 3 und dem Schafteil 2 über die mit der Vorspannkraft der Dehnschaftschraube 11 gegeneinander verspannbaren Abstützflächen 2c, 3c.

## Patentansprüche

1. Bausatz für eine modulare Femurkopfprothese, insbesondere für eine Reoperationsprothese, enthaltend einen in einem Femurknochen (1) verankerbaren distalen Schaftteil (2) und einen auf dessen proximaler Endpartie (2a) mittels einer Schraubverbindung befestigbaren, mit einer künstlichen Gelenkkugel (10) bestückbaren Halsteil (3), wobei die proximale Endpartie (2a) des Schaftteils (2) und der Halsteil (3) mit quer zur Längsachse (L) des Schaftteils (2) gestellten, gegeneinander abstützbaren Abstützflächen (2c bzw. 3c), sowie mit in Richtung der Längsachse (L) orientierten, formschlüssig zusammenführbaren Führungsflächen (2d bzw. 3d) ausgeführt sind, und wobei die Schraubverbindung zum Verbinden von Schaftteil (2) und Halsteil (3) eine mit einer vorbestimmten Vorspannkraft elastisch vorspannbare Dehnschaftschraube (11) enthält, dadurch gekennzeichnet, dass die Dehnschaftschraube (11) mit zwei an örtlich verdickten Führungsabschnitten (13b und 13d; 13e und 13f) des Schraubenschafts (13; 13') ausgebildeten zylindrischen Passflächen ausgeführt ist, welche durch einen mit einem geringeren Querschnitt als die Führungsabschnitte (13b und 13d; 13e und 13f) ausgeführten Dehnabschnitt (13c) des Schraubenschafts (13;13') voneinander getrennt sind und welche zum Zusammenwirken mit zwei entsprechenden, in Bohrungen (14,15) des Halsteils (3) und des Schaftteils (2) ausgebildeten Zentrierflächen bestimmt sind, wobei die eine Zentrierfläche in einem Bohrungsabschnitt (14d;15b) gebildet ist, der vollständig in einem der beiden Teile, Halsteil (3) oder Schaftteil (2), angeordnet ist und die andere Zentrierfläche in Bohrungsabschnitten (14b,15b) gebildet ist, die in beiden Teilen - Halsteil (3) und Schaftteil (2) - angeordnet sind, und zwar in aneinander anschliessenden Bohrungsabschnitten (14b,15b) in der proximalen Endpartie (2a) des Schaftteils (2) und dem daran angrenzenden Abschnitt des Halsteils (3).

2. Bausatz nach Anspruch 1, mit einem an der proximalen Endpartie (2a) des Schaftteils (2) ausgebildeten, zapfenartigen Fortsatz und einer am Halsteil (3) ausgebildeten, zur Aufnahme dieses Fortsatzes (2b) bestimmten Aussparung (3b), wobei die zusammenführbaren Führungsflächen (2d,3d) je am Fortsatz (2b) des Schaftteils (2) und in der Aussparung (3b) des Halsteils (3) ausgebildet sind.

3. Bausatz nach Anspruch 1 oder 2, dadurch gekennzeichnet, dass die proximale Endpartie (2a) des Schaftteils (2) und zumindest eine auf diese aufsetzbare Anschlusspartie (3a) des Halsteils (3) je mit einem zumindest annähernd rechteckförmigen Querschnitt ausgeführt sind, dessen grössere Längenabmessung (A) im wesentlichen in lateraler Richtung des Femurknochens (1) verläuft.

4. Bausatz nach Anspruch 2 oder 3, dadurch gekennzeichnet, dass der Fortsatz (2b) des Schaftteils (2) und die ihn aufnehmende Aussparung (3b) im Halsteil (3) je mit einem zumindest annähernd rechteckförmigen Querschnitt ausgeführt sind.

5. Bausatz nach einem der vorangehenden Ansprüche, gekennzeichnet durch mindestens einen an eine Seitenwand des Halsteils (3), lateral und/oder posterior und/oder anterior, ansetzbaren und am Halsteil (3) befestigbaren Füllkörper (17, 18) als Ersatz für eine fehlende Knochenpartie.

6. Bausatz nach Anspruch 5, dadurch gekennzeichnet, dass der Halsteil (3), oder der Füllkörper (17, 18), mit mindestens einer im wesentlichen in Richtung der Längsachse (L) des Schaftteils (2) verlaufenden, z.B. schwalbenschwanzförmig ausgebildeten, schienenartigen Führungspartie (16) ausgeführt ist und dass der Füllkörper (17, 18), bzw. der Halsteil (3), mit einer entsprechenden nutenartigen Führungsbahn (21) ausgeführt ist.

7. Bausatz nach Anspruch 5 oder 6, dadurch gekennzeichnet, dass der Füllkörper (17, 18) mit einer im wesentlichen in Richtung der Längsachse (L) des Schaftteils (2) auf eine proximale Endpartie des Halsteils (3) aufsetzbaren Kragenpartie (20) ausgeführt ist.

8. Bausatz nach Anspruch 7, dadurch gekennzeichnet, dass der Halsteil (3) in seiner proximalen Endpartie mit einer Vertiefung (9) ausgeführt ist, die zur Aufnahme des Kopfs (12) der Dehnschaftschraube (11) und/oder zumindest eines Teils der Kragenpartie (20) des Füllkörpers (17, 18) bestimmt ist und dass ein diese Vertiefung (9) überdeckendes, gegen die Kragenpartie (20) verspannbares Abdeckelement (22) vorgesehen ist.

9. Femurkopfprothese aus einem Bausatz nach einem der vorangehenden Ansprüche.

## Claims

1. Construction kit for a modular femur head prosthesis, in particular a reoperation prosthesis, including a distal shaft part (2) which can be anchored in a femur bone (1) and a neck part (3) which can be equipped with an artificial joint ball (10) and which can be fixed by means of a screw connection to the proximal end portion (2a) of the shaft part (2), wherein the proximal end portion (2a) of the shaft part (2) and of the neck part (3) are provided with supporting surfaces (2c and 3c respectively) which can be braced against one another and are positioned transverse to the longitudinal axis (L) of the shaft part (2), and also with guide surfaces (2d and 3d respectively) orientated in the direction of the longitudinal axis (L) which can be brought together in a form-fitted manner; and wherein the screw connection includes an extensible shaft screw (11) which can be elastically prestressed with a predetermined bias force, characterised in that the extensible shaft screw (11) is provided with two cylindrical fitted surfaces formed at locally thickened guide sections (13b and 13d; 13e and 13f) of the screw shaft (13; 13') which are separated from one another by an extensible section (13c) of the screw shaft (13; 13') formed with a smaller cross-section than the guide sections (13b and 13d; 13e and 13f) and which are intended to cooperate with two corresponding centering surfaces formed in bores (14, 15) of the neck part (3) and of shaft part (2), with one of the centering surfaces being formed in a bore section (14d; 15b) fully arranged in one of the two parts, neck part (3) or shaft part (2), and with the other centering surface being formed in bore sections (14b, 15b), which are arranged in both parts - neck part (3) and shaft part (2) - and indeed in adjoining bore sections (14b, 15b) in the proximal end portion (2a) of the shaft part (2) and in the adjoining portion of the neck part (3).

2. Construction kit according to claim 1, including a spigot-like projection formed on the proximal end portion (2a) of the shaft part (2) and a recess (3b) defined to receive this projection (2b) formed on the neck part (3), with the guide surfaces (2d, 3d) which can be brought together being respectively formed on the projection (2b) of the shaft part (2) and in the recess (3b) of the neck part (3).

3. Construction kit according to claim 1 or 2, characterised in that the proximal end portion (2a) of the shaft part (2) and at least one connecting portion (3a) of the neck part (3) mountable thereon are each executed with an at least approximately rectangular cross-section the greater length dimension (A) of which extends substantially in the lateral direction of the femur (1).

4. Construction kit according to claim 2 or 3, characterised in that the projection (2b) of the shaft part (2) and the recess (3b) in the neck part which receives the projection are executed with an at least approximately rectangular cross-section.

5. Construction kit according to one of the preceding claims, characterised by at least one filling body (17, 18) as a replacement for a missing piece of bone and mountable laterally of and/or posterior to and/or anterior to a side wall of the neck part (3) and fixable to the neck part (3).

6. Construction kit according to claim 5, characterised in that the neck part (3), or the filling body (17, 18), is provided with at least one rail-like guide section (16), e.g. formed in the shape of a dove-tail, and extending substantially in the direction of the longitudinal axis (L) of the shaft part (2), and in that the filling body (17, 18), or the neck part (3) respectively, is provided with a corresponding groove-like guideway (21).

7. Construction kit according to claim 5 or claim 6, characterised in that the filling body (17, 18) is provided with a collar portion (20) to be mounted substantially in the direction of the longitudinal axis (L) of the shaft part (2) over a proximal end portion of the neck part (3).

8. Construction kit according to claim 7, characterised in that the neck part (3) is provided in its proximal end portion with a recess (9) intended to receive the head (12) of the extensible shaft screw (11) and/or at least a part of the collar portion (20) of the filling body (17, 18); and in that a cover element (22) covering this recess (9) is provided and can be clamped against the collar portion (20).

9. Femur head prosthesis from a construction kit according to one of the preceding claims.

## Revendications

1. Jeu de construction pour une prothèse de tête fémorale modulaire, en particulier une prothèse de ré-opération, comportant une partie de tige distale (2) pouvant être ancrée dans un os de fémur (1) et une partie de col (3) pouvant être fixée sur la partie d'extrémité proximale (2a) de celle-ci par un assemblage par vissage, pouvant être munie d'une boule d'articulation artificielle (10), la partie d'extrémité proximale (2a) de la partie de tige (2) et la partie de col (3) étant réalisées avec des surfaces d'appui (2c, 3c) disposées transversalement à l'axe longitudinal (L) de la partie de tige (2), s'appuyant l'une contre l'autre, et avec des surfaces de guidage (2d, 3d) orientées dans la direction de l'axe longitudinal (L), pouvant être réunies par concordance des formes, et où l'assemblage par vissage pour assembler la partie de tige (2) et la partie de col (3) comporte une vis de tige d'extension (11) pouvant être précontrainte élastiquement avec une force de précontrainte prédéterminée, caractérisé en ce que la vis de tige d'extension (11) est réalisée avec deux surfaces d'ajustage cylindriques réalisées à des tronçons de guidage plus épais (13b, 13d ; 13e, 13f) de la tige de vis (13 ; 13') qui sont séparées par un tronçon d'extension (13c) de la tige de vis (13 ; 13') réalisé en une section transversale plus petite que les tronçons de guidage (13b, 13d ; 13e, 13f) et qui sont prévues pour coopérer avec deux surfaces de centrage correspondantes réalisées dans des perçages (14, 15) de la partie de col (3) et de la partie de tige (2), une surface de centrage étant réalisée dans un tronçon de perçage (14d ; 15b) qui est disposé complètement dans l'une des deux parties, partie de col (3) ou partie de tige (2) et l'autre surface de centrage est réalisée dans des tronçons de perçage (14b, 15b) qui sont disposés dans les deux parties, partie de col (3) et partie de tige (2), et cela dans des tronçons de perçage (14b, 15b) qui se font suite dans la partie d'extrémité proximale (2a) de la partie de tige (2) et dans le tronçon avoisinant celle-ci de la partie de col (3).

2. Jeu de construction selon la revendication 1, avec un prolongement en forme d'ergot réalisé à la partie d'extrémité proximale (2a) de la partie de tige (2) et un évidement (3b) réalisé à la partie de col (3), prévu pour la réception de ce prolongement (2b), les surfaces de guidage aptes à être réunies (2d, 3d) étant réalisées respectivement au prolongement (2b) de la partie de tige (2) et dans la partie de prolongement (3b) de la partie de col (3).

3. Jeu de construction selon la revendication 1 ou 2, caractérisé en ce que la partie d'extrémité proximale (2a) de la partie de tige (2) et au moins une partie de raccordement (3a) de la partie de col (3) pouvant être placée sur celle-ci sont réalisées en une section transversale au moins approximativement rectangulaire dont la dimension longitudinale plus grande (A) s'étend essentiellement dans la direction latérale de l'os de fémur (1).

4. Jeu de construction selon la revendication 2 ou 3, caractérisé en ce que le prolongement (2b) de la partie de tige (2) et l'évidement (3b) dans la partie de col (3) recevant celui-ci sont réalisés respectivement en une section transversale au moins approximativement rectangulaire.

5. Jeu de construction selon l'une des revendications précédentes, caractérisé par au moins un corps de remplissage (17, 18) pouvant être appliqué à une paroi latérale de la partie de col (3) latéralement et/ou postérieurement et/ou antérieurement et pouvant être fixé à la partie de col (3), pour remplacer une partie osseuse manquante.

6. Jeu de construction selon la revendication 5, caractérisé en ce que la partie de col (3) où le corps de remplissage (17, 18) est réalisé avec au moins une partie de guidage (16) en forme de rail, s'étendant essentiellement en direction de l'axe longitudinal (L) de la partie de tige (2), réalisée par exemple en forme de queue d'aronde, et en ce que le corps de remplissage (17, 18) respectivement la partie de col (3) est réalisée avec une voie de guidage (21) en forme de rainure correspondante.

7. Jeu de construction selon la revendication 5 ou 6, caractérisé en ce que le corps de remplissage (17, 18) est réalisé avec une partie de col (20) pouvant être placée essentiellement dans la direction de l'axe longitudinal (L) de la partie de tige (2) sur une partie d'extrémité proximale de la partie de col (3).

8. Jeu de construction selon la revendication 7, caractérisé en ce que la partie de col (3) est réalisée dans sa partie d'extrémité proximale avec un creux (9) qui est prévu pour la réception de la tête (12) de la vis de tige d'extension (11) et/ou au moins d'une partie de la partie de col (20) du corps de remplissage (17, 18) et en ce qu'il est prévu un élément de recouvrement (22) recouvrant ce creux (9), pouvant être tendu contre la partie de col (20).

9. Prothèse de tête fémorale à partir d'un jeu de construction selon l'une des revendications précédentes.
